(19) Europäisches Patentamt
European Patent Office
Office européen des brevets

(11) Veröffentlichungsnummer: **0 162 347**
**B1**

# (12) EUROPÄISCHE PATENTSCHRIFT

(45) Veröffentlichungstag der Patentschrift: **09.12.87**

(51) Int. Cl.[4]: **C 07 C 119/045**

(21) Anmeldenummer: **85105233.2**

(22) Anmeldetag: **30.04.85**

(54) **Neue cycloaliphatische Triisocyanate, ein Verfahren zu ihrer Herstellung und ihre Verwendung bei der Herstellung von Polyurethankunststoffen.**

(30) Priorität: **12.05.84 DE 3417683**

(43) Veröffentlichungstag der Anmeldung: **27.11.85 Patentblatt 85/48**

(45) Bekanntmachung des Hinweises auf die Patenterteilung: **09.12.87 Patentblatt 87/50**

(84) Benannte Vertragsstaaten: **DE FR GB IT NL**

(56) Entgegenhaltungen:
**EP - A - 0 046 917**
**EP - A - 0 104 531**
**EP - A - 0 104 551**
**DE - A - 1 932 832**
**FR - A - 1 447 964**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Knöfel, Hartmut, Dr., Dülmener Weg 21, D-5068 Odenthal (DE)**
Erfinder: **Penninger, Stefan, Dr., Hahnenstrasse 106, D-5024 Pulheim (DE)**
Erfinder: **Brockelt, Michael, Neuenhauser Weg 1, D-5060 Bergisch-Gladbach 2 (DE)**
Erfinder: **Hammen, Günter, Dr., Bunzlauer Weg 13, D-4049 Rommerskirchen 1 (DE)**
Erfinder: **Stutz, Herbert, Dr., Schulstrasse 81, D-4047 Dormagen 5 (DE)**

**EP 0 162 347 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue cycloaliphatische Triisocyanate, ein Verfahren zu ihrer Herstellung durch Phosgenierung der ihnen zugrundeliegenden Triamine und ihre Verwendung als Ausgangsmaterial zur Herstellung von Polyurethankunststoffen nach dem Polyadditionsverfahren, insbesondere auf dem Gebiet der Lacke und Beschichtungen.

Aliphatische und cycloaliphatische Diisocyanate wie z.B. 4,4'-Diisocynatodicyclohexylmethan, 3-Isocyanatomethyl-3,5,5-trimethyl-cyclohexylisocyanat und Hexamethylendiisocyanat werden grosstechnisch zur Herstellung von lichtechten Beschichtungsmaterialien mit ausgezeichneter Witterungsbeständigkeit verwendet [vgl. H. Wagner, H.F. Sarx, Lackkunstharze, 5. Auflage, Carl Hanser Verlag München (1071), 153 ff., H. Kittel, Lehrbuch der Lacke und Beschichtungen, Verlag W.A. Colomb in der Hennemann GmbH Berlin-Oberschwandorf (1973)].

Da aber niedermolekulare Isocyanate einen hohen Dampfdruck besitzen und gegebenenfalls toxisch wirken, werden sie vor ihrer Anwendung modifiziert, z.B. trimerisiert, biuretisiert oder durch Umsetzung mit niedermolekularen Polyolen präpolymerisiert, so dass sie bei der Weiterverarbeitung den gewerbehygienischen Anforderungen genügen. Durch diese Modifizierung entstehen höhermolekulare Verbindungen mit höherer Funktionalität, die einen praktisch vernachlässigbaren Dampfdruck aufweisen (vgl. z.B. Kunststoff-Handbuch von G.W. Becker und D. Braun, Carl Hanser Verlag München Wien (1983), Band 7, «Polyurethane», Seiten 541 bis 543).

Die nachträgliche Modifizierung der monomeren Diisocyanate ist allerdings mit mehreren Nachteilen behaftet. So stellt der zusätzliche Schritt der Modifizierung einen erhöhten, mit zusätzlichen Kosten verbundenen Aufwand dar, zumal in den meisten Fällen eine technisch aufwendige Abtrennung des modifizierten Produkts von überschüssigen Monomeren erforderlich ist. Ausserdem können in gewissen Fällen im Verlauf der Lagerung der modifizierten Polyisocyanate monomere Diisocyanate durch Rückspaltung gebildet werden. Die modifizierten Polyisocyanate sind im übrigen in der Regel höherviskos und eignen sich nicht oder nur bedingt für eine lösungsmittelarme bzw. lösungsmittelfreie Anwendung. Bedingt durch ihren niedrigen Dampfdruck sind sie ausserdem nicht durch Destillation zu reinigen.

Die EP-A-0 104 531 und die EP-A-0 104 551 beschreiben zwar bereits Diisocyanate, die den nachstehend näher beschriebenen erfindungsgemässen Triisocyanaten in struktureller Hinsicht weitgehend entsprechen, jedoch sind die Diisocyanate wegen ihrer Difunktionalität als Lackpolyisocyanat weit weniger gut geeignet als die erfindungsgemässen Triisocyanate. In den genannten Vorveröffentlichungen fehlt auch jeglicher Hinweis auf die Herstellbarkeit von Triisocyanaten der erfindungsgemässen Art bzw. deren technisch fortschrittlichen Eigenschaften.

Die DE-A-1 932 832 befasst sich mit einem cycloaliphatischen Triisocyanat, welches als Lackpolyisocyanat keine praktische Bedeutung erlangte, was insbesondere darauf zurückzuführen sein dürfte, dass das Triisocyanat dieser Vorveröffentlichung einen deutlich niedrigeren Isocyanatgehalt bei einer ganz wesentlich erhöhten Viskosität aufweist und im übrigen nicht destillativ gereinigt werden kann.

Es war daher die der Erfindung zugrundeliegende Aufgabe, neue Polyisocyanate zur Verfügung zu stellen, die auf einfache Weise zugänglich sind und auch in unmodifizierter Form die Vorteile der genannten Lackpolyisocyanate des Standes der Technik in sich vereinigen, ohne mit deren Nachteilen behaftet zu sein.

Diese Aufgabe konnte durch die Bereitstellung der nachstehend näher beschriebenen erfindungsgemässen Polyisocyanate und des zu ihrer Herstellung geeigneten Verfahren gelöst werden.

Gegenstand der Erfindung sind gegebenenfalls Stellungs- und/oder Stereoisomerengemische darstellende Triisocyanate der Formel (I)

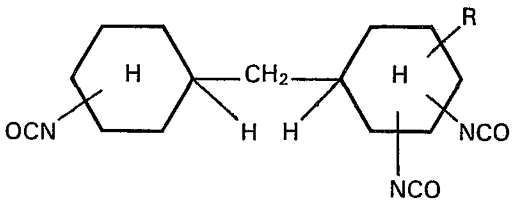
(I)

in welcher

R für Wasserstoff oder einen Alkylrest mit 1 bis 4 Kohlenstoffatomen vorzugsweise für Wasserstoff oder eine Methylgruppe steht.

Gegenstand der Erfindung ist auch ein Verfahren zur Herstellung der erfindungsgemässen Triiscocyanate, welches dadurch gekennzeichnet ist, dass man die ihnen zugrundeliegenden Triamine in an sich bekannter Weise phosgeniert.

Gegenstand der Erfindung ist schliesslich auch die Verwendung der erfindungsgemässen Triisocyanate als Aufbaukomponente bei der Herstellung von Polyurethankunststoffen nach dem Isocyanat-Polyadditionsverfahren.

Ausgangsmaterialien für das erfindungsgemässe Verfahren sind die den erfindungsgemässen Triisocyanaten entsprechenden Triamine der Formel

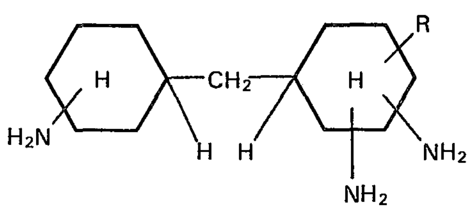
(II)

in welcher

R die bereits genannte Bedeutung hat.

Ausgangsmaterialien für die Herstellung der beim erfindungsgemässen Verfahren einzusetzenden Polyamine sind die entsprechenden aromatischen Triamine der Formel (III)

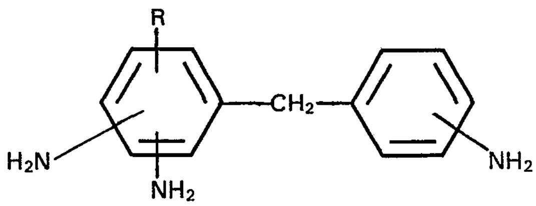

(III)

in welcher

R die bereits gennante Bedeutung hat.

Die Herstellung dieser aromatischen Triamine erfolgt in an sich bekannter Weise beispielsweise durch Umsetzung von N-[4(2)-Aminobenzyl]-anilin mit gegebenenfalls Alkyl-substituierten Phenylendiaminen gemäss Beilstein 13 H, Seite 309 bzw. gemäss DE-PS 107 718 oder durch Umsetzung (i) eines 3- oder 4-Nitrobenzylhalogenids, von Benzylalkohol oder eines Nitrobenzylchlorid-Isomerengemischs mit (ii) Nitrobenzol, Alkyl-substituiertem Nitrobenzol, Alkylbenzol oder Benzol in Gegenwart eines Friedel-Crafts- oder eines Säurekatalysators, Nitrierung der so erhaltenen Umsetzungsprodukte zu den entsprechenden Trinitroverbindungen und Hydrierung der Nitrogruppen in Analogie zu EP-A-46 917. Die nach der letztgenannten Methode erhaltenen Trinitroverbindungen und die hieraus hergestellten aromatischen Triamine stellen, herstellungsbedingt, technische Gemische dar, die neben den entsprechenden trifunktionellen Verbindungen noch difunktionelle Verbindungen enthalten können. Aus diesen technischen Gemischen kann jedoch der Anteil an difunktionellen Verbindungen gewünschtenfalls auf der Aminstufe destillativ entfernt werden.

Typische Beispiele geeigneter Ausgangsmaterialien sind

2,4,4'(2')-Triaminodiphenylmethan,
2,6,4'(2')-Triaminodiphenylmethan,
4,6,4'(2')-Triamino-3-methyl-diphenylmethan,
2,6,4'(2')-Triamino-3-methyl-diphenylmethan,
3,5,4'(2')-triamino-4-methyl-diphenylmethan,
2,6,4'(2')-Triamino-4-methyl-diphenylmethan,
3,5,4'(2')-Triamino-2-methyl-diphenylmethan,
4,6,4'(2')-Triamino-2-methyl-diphenylmethan,

oder deren Gemische. Auch die entsprechenden Ethyl-, Isopropyl-, n-Propyl- oder m-, iso- oder tert.-Butylsubstituierten Triamino-diphenylmethane können eingesetzt werden.

Bevorzugte Ausgangsmaterialien sind jedoch Isomerengemische von Methyl-substituierten Triamino-diphenylmethanen oder deren technischen Gemische mit den entsprechenden Diaminen, wie sie bei der Trinitrierung von 2- und/oder 4-Methyldiphenylmethan bzw. von aus diesen Isomeren bestehenden Kohlenwasserstoffgemischen, Reduktion der Nitrogruppen und gegebenenfalls anschliessender destillativer Aufarbeitung anfallen. Die besonders bevorzugten, nach dieser Verfahrensweise hergestellten Ausgangsmaterialien bestehen im allgemeinen zu über 80 Gew.-% aus Triaminodiphenylmethanen, die ihrerseits zu über 90 Gew.-% aus Aminobenzyl-diaminotoluol-Isomeren bestehen.

Wie oben ausgeführt, kann es sich bei den Ausgangsmaterialien ausschliesslich um aromatische Triamine der Formel III bzw. und Gemische derartiger Triamine oder aber um Gemische derartiger Triamine mit den entsprechenden Diaminen handeln, welche vorzugsweise an jedem aromatischen Ring eine Aminogruppe aufweisen. In diesen Gemischen können bis zu 90, vorzugsweise bis zu 50 und insbesondere bis zu 20 Gew.-%, bezogen auf Gesamtgemisch, an solchen Diaminen vorliegen. Die herstellungsbedingt einen hohen Gehalt an derartigen aromatischen Diaminen aufweisenden Polyamingemische können gegebenenfalls vor der Herstellung der erfindungsgemäss einzusetzenden Ausgangsmaterialien durch Hydrierung der aromatischen Polyamine ganz oder weitgehend destillativ von den Diaminen befreit werden. Es ist jedoch auch möglich, die aromatischen Polyamingemische als solche der Hydrierung zu unterziehen und gegebenenfalls die hydrierten erfindungsgemäss als Ausgangsmaterialien einzusetzenden Polyamingemische von den Diaminen und sonstigen, bei der Hydrierung anfallenden Nebenprodukten zu befreien. Schliesslich ist es ebenfalls möglich, nach der Durchführung des erfindungsgemässen Verfahrens eine destillative Abtrennung von difunktionellen Verfahrensprodukten (erhalten durch Phosgenierung der entsprechenden Diamine) und gegebenenfalls anderen Nebenprodukten durchzuführen um zu weitgehend reinen Polyisocyanaten der Formel (I) zu gelangen.

Die Kernhydrierung der aromatischen Polyamine erfolgt im allgemeinen nach den bekannten Methoden des Standes der Technik (vgl. P. Rylander, Catalytic Hydrogenation in Organic Syntheses, Academic Press New York, San Francisco, London (1979), Seite 190). Hierbei werden die aromatischen Amine bis zur vollständigen Wasserstoffaufnahme katalytisch hydriert. Die Hydrierung erfolgt bei 20 bis 300°C unter einem Druck von 100 bis 300 bar insbesondere von 150 bis 250°C und bei einem Druck von 70 bis 300 bar, insbesondere 120 bis 300 bar.

Die Hydrierung erfolgt in Gegenwart von 0,1 bis 30 Gew.-%, vorzugsweise von 0,1 bis 10 Gew.-%, bezogen auf katalytisch wirksames Metall einerseits und Diaminoverbindung andererseits, eines Hydrierungskatalysators. Geeignete Katalysatoren sind beispielsweise, gegebenenfalls auf inerten Trägern wie Aktivkohle, Kieselgel, Calciumcarbonat, Bariumsulfat und insbesondere Aluminiumoxid vorliegende Elemente der 8. Nebengruppe des Periodensystems der Elemente oder katalytisch wirksame anorganische Verbindungen dieser Elemente.

Besonders gut geeignet sind z.B. Ruthenium-, Platin-, Rhodium-, Nickel- und/oder Kobaltkatalysatoren in elementarer oder chemisch gebundener Form. Besonders bevorzugt werden Ruthenium oder katalytisch wirksame Rhutheniumverbindungen eingesetzt. Beispiele geeigneter Rutheniumverbindungen sind Rutheniumoxid, Bariumperruthenit, Natrium-, Kalium-, Silber-, Calcium- oder Magnesiumruthenat, Natriumperruthenat, Rutheniumpentafluorid, Rutheniumtetrafluoridhydrat oder Rutheniumtrichlorid. Falls Trägersubstanzen für die Katalysatoren mitverwendet werden, beträgt der Metallgehalt des Trägerkatalysators im allgemeinen 1 bis 10 Gew.-%, vorzugsweise 1 bis 5 Gew.-%. Im übrigen ist selbstverständlich die Art und Menge des einzusetzenden Katalysators keineswegs wesentlich.

Es ist oft zweckmässig, die Hydrierungsreaktion in Gegenwart von Ammoniak durchzuführen, da durch die Anwesenheit von Ammoniak unerwünschte Desaminierungsreaktionen und die Bildung von sekundären Aminen als Nebenprodukte unterdrückt werden können. Falls Ammoniak mitverwendet wird, geschieht dies in Mengen von 0,1 bis 30 Gew.-%, vorzugsweise 5 bis 10 Gew.-%, bezogen auf zu hydrierende Ausgangsmaterialien.

Die Hydrierung kann lösungsmittelfrei oder in Gegenwart inerter Lösungsmittel durchgeführt werden. Im allgemeinen werden niedrigschmelzende bzw. flüssige aromatische Amine in Substanz, hochschmelzende Diamine dagegen in gelöster Form hydriert. Als Lösungsmittel eignen sich unter den Reaktionsbedingungen inerte organische Verbindungen mit niedrigem Siedepunkt, vorzugsweise Alkohole wie Methanol, t-Butanol, Ethanol, n-Propanol, i-Propanol oder Ether wie z.B. Dioxan, Tetrahydrofuran oder Diethylether oder Kohlenwasserstoffe wie Cyclohexan.

Die Durchführung der Hydrierung findet kontinuierlich in einem Reaktionsrohr, einer Druckkesselkaskade oder vorzugsweise diskontinuierlich in einem Rührautoklaven statt, indem man den Autoklaven mit Katalysator, der zu hydrierenden Substanz und gegebenenfalls einem Lösungsmittel beschickt, mehrmals mit Inertgas spült und gegebenenfalls Ammoniak zudosiert. Danach drückt man Wasserstoff auf, bringt das Gemisch auf Reaktionstemperatur, hydriert bis Druckkonstanz erreicht ist und rührt während eines weiteren Zeitraums von ca. 0,5 bis 5 Stunden bei gleicher Temperatur. Nach Abkühlung des Reaktionsgemisches und Abtrennung des Katalysators wird das Hydrierprodukt im allgemeinen destillativ aufgearbeitet.

Die Hydrierungsprodukte fallen in hohen Ausbeuten an und lassen sich, wie ausgeführt, erforderlichenfalls destillativ von Nebenprodukten befreien. Auch nach einer derartigen destillativen Reindarstellung der Triamine stellen diese im allgemeinen Gemische von Stereo- und gegebenenfalls Stellungsisomeren dar. Durch die genannte destillative Aufarbeitung sind Triamine zugänglich, die zu über 80, vorzugsweise zu über 90 Gew.-% aus Triaminen bestehen, die der allgemeinen Formel (II) entsprechen. Jedoch auch die nicht destillativ aufgearbeiteten Hydrierungsprodukte stellen Triamine der Formel (II) dar, die jedoch nicht nur Stellungs- und/oder Stereoisomerengemische darstellen können, sondern darüber hinaus im Gemisch mit bis zu 90, vorzugsweise bis zu 50 und insbesondere bis zu 40 Gew.-% an anderen, gegebenenfalls alkylsubstituierten Di- und/oder Triaminen mit Diphenylmethan-, Benzyl-cyclohexan- oder Dicyclohexylmethan-Struktur vorliegen. Die genannten Diamine können dann vorliegen, wenn als Ausgangsmaterialien aromatische Triamine verwendet werden, die, wie oben ausgeführt, aromatische Diamine enthalten und/oder wenn es während der Hydrierungsreaktion im geringen Umfang zu einer Desaminierung des cycloaliphatischen Rings kommt. Eine derartiger Desaminierung kann jedoch, wie oben ausgeführt, gegebenenfalls durch Mitverwendung von Ammoniak bei der Hydrierungsreaktion zurückgedrängt werden. Auch Monoamine können in ganz geringen Mengen durch Desaminierung entstehen. Sie können jedoch sehr leicht destillativ entfernt werden. Polyamine mit Diphenylmethan- oder Benzyl-cyclohexan-Struktur liegen allenfalls bei nicht vollständig ablaufender Hydrierung in geringen Mengen in den Hydrierungsprodukten vor und beeinträchtigen im allgemeinen deren weitere Verwendbarkeit nicht.

Im allgemeinen ist eine destillative Reindarstellung der Hydrierungsprodukte vor ihrer Verwendung beim erfindungsgemässen Verfahren nicht erforderlich, da, wie bereits ausgeführt, gegebenenfalls eine destillative Aufarbeitung der erfindungsgemässen Verfahrensprodukte nach der Phosgenierungsreaktion durchgeführt werden kann. Ebensowenig kommt es bezüglich der Verwendbarkeit der Hydrierungsprodukte für das erfindungsgemässe Verfahren auf deren Stellungs- und Stereoisomerie bzw. auf die entsprechende Isomerenverteilung an.

Typische Beispiele von geeigneten Ausgangspolyaminen für das erfindungsgemässe Verfahren sind die den obengenannten aromatischen Triaminen entsprechenden cycloaliphatischen Triamine, die, wie gesagt, gewünschtenfalls von Nebenprodukten, wie sie bei der Hydrierungsreaktion oftmals entstehen, destillativ befreit werden können. Von dieser Einschränkung abgesehen, entsprechen die beim erfindungsgemässen Verfahren einzusetzenden Ausgangspolyamine den zu ihrer Herstellung eingesetzten, oben beispielhaft genannten aromatischen Polyaminen bzw. Polyamingemischen.

Bei der Durchführung des erfindungsgemässen Verfahrens zur Herstellung der neuen Triisocyanate erfolgt die Phosgenierung der Ausgangsamine oder deren Salze nach an sich bekannten Verfahren in Gegenwart eines inerten organischen Lösungsmittels (vgl. Houben-Weyl, Methoden der organischen Chemie, Georg Thieme Verlag Stuttgart (1952), Band 8, 4. Auflage, Seiten 120 ff). Unter «Ausgangsaminen» sind in diesem Zusammenhang, den oben gemachten Ausführungen entsprechend, sowohl reine, gegebenenfalls Stellungs- und Stereoisomerengemische darstellende Verbindungen der allgemeinen Formel (II) als auch deren Gemische mit bis zu 90, vorzugsweise bis zu 50 und insbesondere bis zu 40 Gew.-% an anderen, gegebenenfalls Alkylsubstituierten Di- und/oder Triaminen mit Diphenylmethan-, Benzyl-cyclohexyl- oder Dicyclohexylmethan-Struktur zu verstehen, wobei sich diese Prozentangaben jeweils auf das Gesamtgemisch beziehen.

Als zu phosgenierende Salze eignen sich vorzugsweise Hydrochloride oder Ammoniumcarbamate, die durch Sättigung der Polyaminlösungen mit gasförmigem Chlorwasserstoff bzw. Kohlendioxid anfallen. Prinzipiell können auch andere Salze, die beispielsweise durch Neutralisation der Polyamine mit Protonen abspaltenden Säuren anfallen, phosgeniert werden.

Die Selektivität der Phosgenierungsreaktion ist weitgehend von der Aminkonzentration und vom Phosgenüberschuss abhängig. Vorzugsweise werden Phosgen in einem hohen molaren Überschuss und die zu phosgenierenden Amine in stark verdünnter Form eingesetzt. Im allgemeinen beträgt der molare Phosgenüberschuss 100 bis 2000%, vorzugsweise 100 bis 1000% und die Aminkonzentration, bezogen auf die Gesamtmenge an Amin einerseits und Lösungsmittel andererseits, 0,1 bis 15 Gew.-%, vorzugsweise 5 bis 10 Gew.-%.

Als Lösungsmittel können beliebige inerte organische Flüssigkeiten oder deren Gemische mit einem Siedepunkt von 60 bis 250°C, d.h. Halogenkohlenwasserstoffe, Aromaten, Hydroaromaten sowie deren Chlorverbindungen verwendet werden. Als Beispiele geeigneter Lösungsmittel seien Xylol, Mesitylen, Chlorbenzol, Dichlorbenzol, Trichlorbenzol, Chlornaphthalin und Dichlorethan genannt.

Die Reaktion wird entweder einstufig durch Heissphosgenierung bei Temperaturen von 100 bis 250°C oder zweistufig durch Kalt/Heissophosgenierung bei Temperaturen von −20 bis 250°C unter Normaldruck durchgeführt.

Bei Verwendung der freien Amine als Ausgangsverbindung (Basenphosgenierung) wird zuerst bei Temperaturen von −20 bis +60°C Ammoniumcarbamidsäurechlorid hersgestellt, das dann bei Temperaturen von 20 bis 250°C mit Phosgen zum Polyisocyanat weiterreagiert.

Die Reinigung der Verfahrensprodukte erfolgt im allgemeinen nach Entphosgenierung durch Abdampfen des Lösungsmittels und anschliessende Destillation unter vermindertem Druck.

Die erfindungsgemässen Verfahrensprodukte, d.h. die neuen erfindungsgemässen Triisocyanate fallen in hohen Ausbeuten als farblose bis gelb gefärbte, niedrigviskose Flüssigkeiten an und stellen wertvolle Aufbaukomponenten bei der Herstellung von Polyurethankunststoffen nach dem Isocyanat-Polyadditionsverfahren dar. Die Stellungs- und/oder Stereoisomerie der neuen Triisocyanate entspricht weitgehend der Isomerie der bei der Phosgenierung eingesetzten Triamine. Im allgemeinen ist es nicht erforderlich, die beim erfindungsgemässen Verfahren anfallenden Gemische in einzelne Stellungs- und/oder Stereoisomere aufzutrennen, da die erfindungsgemässen Verfahrensprodukte ohne derartige Trennungsoperationen direkt der erfindungsgemässen Verwendung zugeführt werden können. Wie bereits ausgeführt können gegebenenfalls im Gemisch mit den erfindungsgemässen Triisocyanaten vorliegende Diisocyanate und sonstige Nebenprodukte (insbesondere Phosgenierungsprodukte von Triaminen mit Diphenylmethan- oder Dicyclohexylmethan-Struktur) destillativ von den erfindungsgemässen Triaminen ganz oder teilweise abgetrennt werden. Für viele Anwendungsgebiete der erfindungsgemässen Triisocyanate ist jedoch eine derartige Reindarstellung nicht erforderlich, da auch die entsprechenden Polyisocyanatgemische, die beispielsweise mindestens 50 Gew.-%, vorzugsweise mindestens 80 Gew.-% an erfindungsgemässen Polyisocyanaten der Formel (I) aufweisen, wertvolle neue Ausgangsmaterialien für die Polyurethanchemie darstellen. Die neuen erfindungsgemässen Triisocyanate bzw. ihre Gemische mit den genannten Nebenprodukten, welche mindestens 50 Gew.-%, vorzugsweise mindestens 80 Gew.-% an erfindungsgemässen Triisocyanaten der Formel (I) enthalten, können besonders vorteilhaft zur Herstellung von Polyurethanlacken, bzw. Beschichtungsmaterialien eingesetzt werden, wobei sie bei den an sich bekannten Verfahren zur Herstellung derartiger Kunststoffe anstelle oder zusammen mit den bisher eingesetzten Polyisocyanaten zum Einsatz gelangen.

Ein besonderer Vorteil der erfindungsgemässen Polyisocyanate gegenüber den bekannten Lackpolyisocyanaten des Standes der Technik ist in dem Umstand zu sehen, dass es sich um niedrigviskose und destillierbare Flüssigkeiten handelt. Die neuen Triisocyanate besitzen dennoch einen ausreichend niedrigen Dampfdruck, so dass sie bei der erfindungsgemässen Verwendung zur Herstellung von Polyurethanen, insbesondere von Polyurethanlacken den gewerbehygienischen Anforderungen genügen.

Die nachfolgenden Beispiele dienen der weiteren Erläuterung der Erfindung. Alle Prozentangaben beziehen sich, soweit nichts anderslautendes vermerkt, auf Gewichtsprozente. Die Analyse der Isomerenverteilung der Zwischen- und Endprodukte erfolgte gaschromatographisch.

*Beispiel 1*

a) In einem 700 ml Rührautoklaven werden 380 g eines technischen Amingemischs bestehend aus 1,4% 4,4'-Diaminodiphenylmethan, 10,2% 4,6,2'-Triamino-3-methyldiphenylmethan, 6,8% 2,6,4'-Triamino-3-methyldiphenylmethan, 78,7% 4,6,4'-Triamino-3-methyldiphenylmethan und 2,9% an anderen aromatischen Polyaminen, das durch Umsetzung von 2,4-Diaminotoluol mit technisch hergestelltem N-Aminobenzyl-anilin mit einem Gehalt an N-[4-Aminobenzyl]-anilin von über 80%, in Gegenwart von Salzsäure (Protonierungsgrad: 50%), anschliessender Neutralisation des Katalysators mit Natronlauge und destillativer Aufarbeitung hergestellt worden war, 300 g tert.-Butanol und 76 g Ruthenium-Aluminiumoxid-Trägerkatalysator (5% Ru) vorgelegt und nach mehrmaligem Spülen mit Stickstoff und Wasserstoff 100 bar Wasserstoff aufgedrückt. Man erhitzt den Autoklaveninhalt zuerst auf 130°C (Schmelzpunkt des Amingemisches) betätigt den Rührer und erhöht die Temperatur auf 200°C. Nun werden bei konstanter Temperatur 275 bar Wasserstoff aufgedrückt, bis nach 5,25 Stunden Druckkonstanz erreicht ist. Man lässt den Autoklaveninhalt auf ca. 60°C abkühlen, entspannt den Autoklaven und nimmt das Rohprodukt in Methanol auf.

Der Katalysator wird abgenutscht und das Produkt destilliert. Bei einer Siedetemperatur von 115 bis 170°C/0,9 mbar gehen 364,4 g eines Amingemisches über, das laut gaschromatographischer Analyse folgende Zusammensetzung besitzt:

30,3% Diamino-methyldicyclohexylmethan (Isomerengemisch)
61,3% Triamino-methyldicyclohexylmethan (Isomerengemisch)
8,4% nichtidentifizierter Amine.

Die genannten Triamine bestehen ausschliesslich aus solchen, die eine Aminogruppe am unsubstituierten Cyclohexanring und zwei Aminogruppen am methylsubstituierten Cyclohexanring aufweisen.

Nach einer weiteren Destillation wurden 164,0 g eines bei 139 bis 170°C/0,1 mbar siedenden Hydrierungsproduktes gewonnen, das nach gaschromagraphischen Befund zu 92,1% aus Triamino-methyldicyclohexylmethan-Isomeren der genannten Art besteht.

b) Man löst 95 g des gemäss a) destillativ gereinigten Hydrierungsprodukts in 700 ml trockenem Chlorbenzol und leitet bei 40 bis 45°C bis zur Sättigung Kohlendioxid ein. Die entstandene Suspension lässt man unter intensivem Rühren bei 0°C in eine Lösung von 250 g Phosgen in 700 ml Chlorbenzol tropfen. Man erhitzt das Reaktionsgemisch unter Einleiten von Phosgen auf Siedetemperatur und kocht 2 Stunden bis der Feststoff vollständig in Lösung gegangen ist. Nun wird weitere 3 Stunden nachphosgeniert, wobei ca. 300 g/h Phosgen eingeleitet werden. Man verteilt überschüssiges Phosgen, indem man bei Siedehitze 1 Stunde Stickstoff in die Lösung bläst, destilliert das Lösungsmittel bei 1000 bis 100 mbar ab und erhält nach Flashdestillation bei 180 bis 230°C/0,3 bis 0,5 mbar 107 g Rohisocyanat. Nach erneuter Destillation werden 105 g Triisocyanato-methyldicyclohexylmethan der Formel (I) (R = $CH_3$) als Isomerengemisch mit einem NCO-Gehalt von 38,6% als bei 165 bis 170°C/0,05 mbar destillierende Fraktion erhalten.

*Beispiel 2*

a) Einen 0,7 l Rührautoklaven beschickt man mit 14 g Ruthenium-Aluminiumoxid-Trägerkatalysator (5% Ru) und 350 g eines Kondensationsproduktes aus N-[4(2)-Aminobenzyl]-anilin und m-Phenylendiamin folgender Zusammensetzung:

| | |
|---|---|
| 2,2% | m-Phenylendiamin |
| 0,6% | 2,6,4'-Triamino-diphenylmethan |
| 14,5% | 2,4,2'-Triamino-diphenylmethan |
| 82,7% | 2,4,4'-Triamino-diphenylmethan |

Nach mehrmaligem Spülen mit Stickstoff und Wasserstoff dosiert man 39 g flüssigen Ammoniaks zu, drückt 100 bar Wasserstoff auf und erhitzt den Autoklaven auf 140°C. Man hydriert nun unter Rühren mit 275 bar Wasserstoff, erhöht die Temperatur innerhalb von 23 Stunden auf 200°C und hydriert weitere 7 Stunden bei gleicher Temperatur. Man lässt den Autoklaven auf 60°C abkühlen, entspannt und trennt den Katalysator durch Filtration über einer Drucknutsche bei 70°C ab. Nach Destillation bei 127 bis 185°C/0,4 mbar erhält man 337,5 g eines Gemisches cycloaliphatischer Amine folgender Zusammensetzung:

| | |
|---|---|
| 2,5% | Monoamino-dicyclohexylmethan (Isomerengemisch) |
| 21,4% | Diamino-dicyclohexylmethan (Isomerengemisch) |
| 74,6% | Triamino-dicyclohexylmethan (Isomerengemisch) |
| 0,6% | 4-(2,4-Diaminobenzyl)-cyclohexylamin |
| 0,9% | nicht identifizierter Amine |

Durch erneute Destillation bei 127 bis 157°C/0,02 bis 0,3 mbar werden 259,3 g eines Gemisches verschiedener Triamino-dicyclohexylmethan-Isomere erhalten, das 8,5% Diamino-dicyclohexylmethan enthält. Bei den Triaminen handelt es sich um solche, die an einem Cyclohexanring zwei Aminogruppen und am anderen Cyclohexanring eine Aminogruppe als Substituenten enthalten.

b) Man löst 112,5 g des gemäss 2a) hergestellten und destillativ gereinigten Hydrierungsprodukts in 1900 ml trockenem Chlorbenzol und leitet Kohlendioxid bis zur vollständige Absorption ein. Es bildet sich eine farblose Suspension, die nach Abkühlen auf 0°C unter Rühren mit 600 g Phosgen begast wird. Unter Einleiten von 150 g/h Phosgen heizt man innerhalb von 3 Stunden auf 110°C auf, und rührt nachdem der Feststoff vollständig in Lösung gegangen ist unter gleichen Bedingungen 4 Stunden nach.

Nach einstündigem Entphosgenieren und Abdestillieren des Lösungsmittels wird das Rohisocyanat bei 180 bis 220°C/0,2 bis 0,5 mbar destilliert. Dabei fallen 130 g Rohprodukt mit einem NCO-Gehalt von 39,1% an. Nach erneuter Destillation bei 174 bis 180°C/0,1 bis 0,4 mbar werden 107 g Triisocyanato-dicyclohexylmethan der Formel (I) (R = H) als Isomerengemisch mit einem NCO-Gehalt von 41% und einer Viskosität bei 25°C von 112 mPa.s isoliert.

*Beispiel 3*

a) 350 g eines aromatischen Polyamingemischs bestehend aus 2,6% Diaminotoluol (Isomerengemisch), 6,2% Diaminomethyldiphenylmethan (Isomerengemisch, in welchem zu über 90% solche Diamine vorliegen, die jeweils an einem aromatischen Ring einen Aminosubstituenten aufweisen) und 91,2% Triaminomethyldiphenmylmethan (Isomerengemisch in welchem zu über 90% solche Triamine vorliegen, die eine Aminogruppe am unsubstituierten aromatischen Ring und zwei Aminogruppen am methylsubstituierten aromatischen Ring aufweisen), und welches durch Nitrierung von Methyldiphenylmethan und anschliessende katalytische Hydrierung unter Verwendung von Raney-Nickel gemäss EP-A 46 917 hergestellt worden war, werden in Gegenwart von 35 g Ruthenium (5%)-Aluminiumoxid-Trägerkatalysator und in Gegenwart von 35 g Ammoniak in einem 0,7 l Rührautoklaven bei 200°C und 275 bar gemäss Beispiel 1 hydriert. Nach zweimaliger Destillation werden 69,2 g eines bei 132 bis 148°C/0,1 mbar siedenden Hydrierprodukts isoliert, das zu 91% aus Triaminomethyldicyclohexylmethan der Formel (I) (R = $CH_3$) und zu 9% aus Diaminomethyldicyclohexylmethan besteht.

b) Man löst 63 g des destillierten Hydrierungsprodukts aus Beispiel 3a) in 400 ml trockenem Chlorbenzol und sättigt die Lösung mit Kohlendioxid.

Die entstandene weisse Suspension von Ammoniumcarbamaten lässt man unter Rühren bei 0 bis 10°C zu einer Lösung von 150 g (1,53 Mol) Phosgen in 400 ml Chlorbenzol zutropfen. Unter Einleiten von Phosgen wird auf Rückflusstemperatur erhitzt bis nach ca. einstündigem Kochen eine klare Lösung entsteht. Man phosgeniert weitere 3 Stunden nach, vertreibt überschüssiges Phosgen durch Einblasen von Stickstoff und erhält nach Abdampfen des Lösungsmittels und Flashdestillation bei 170 bis 200°C/0,1

bis 0,3 mbar 65 g Rohphosgenat, das gemäss gaschromatographischer Analyse zu 90% aus Triisocyanato-methyldicyclohexylmethan besteht. Eine erneute Destillation bei 168 bis 175°C/0,1 mbar liefert 55 g eines Polyisocyanatgemisches, das zu 96,7% aus Triisocyanato-methyldicyclohexylethan der Formel (I) (R = $CH_3$) in Form eines Isomerengemischs und zu 3,3% aus Diisocyanato-methyldicyclohexylmethan-Isomeren besteht und einen NCO-Gehalt von 38,65% und eine Viskosität von 196 mPa.s/25°C aufweist.

*Beispiel 4*

a) In einem 1,3 l Rührautoklaven werden 253 g (1,19 Mol) 2,4,4'-Triamino-diphenylmethan, hergestellt durch Umkristallisation des gemäss Beispiel 2 eingesetzten Ausgangsgemischs in 1,2-Dichlorbenzol, 250 ml tert.-Butanol und 50,6 g des in Beispiel 1 eingesetzten Katalysators vorgelegt. Nach mehrmaligem Spülen mit Stickstoff und Wasserstoff werden 100 bar Wasserstoff aufgedrückt, der Autoklaveninhalt auf 180°C erhitzt und unter intensivem Rühren bei 275 bar hydriert. Nach 105 Minuten ist die Wasserstoffaufnahme beendet. Der Autoklav wird auf 60°C abgekühlt, entspannt und die Lösung vom Katalysator abgesaugt. Nach Abdestillieren des Lösungsmittels wird das Rohprodukt bei 115-195°C/0,5 mbar einer Flashdestillation unterworfen und 238,3 g Destillat gewonnen. Dieses besteht zu 81,5% aus 2,4,4'-Triaminodicyclohexylmethan, 16,1% aus Diamino-dicyclohexylmethan und zum Rest aus undefinierten Polyaminen. Die Reindestillation dieses Produkts ergibt 175,4 g 2,4,4'-Triaminodicyclohexylmethan, das 2,3%, bezogen auf Gesamtgemisch, an Diaminodicyclohexylmethan (Isomerengemisch) enthält und einen Siedepunkt von 147 bis 153°C/0,2 mbar aufweist.

b) 169 g des gemäss Beispiel 4a) destillativ gereinigten Hydrierungsprodukts werden in 2,5 l Chlorbenzol gelöst und daraufhin Kohlendioxid bis zur vollständigen Absorption eingeleitet. Es bildet sich eine Suspension, die auf -20°C gekühlt und mit 400 g Phosgen versetzt wird. Nach 15minütigem Rühren wird der Phosgenstrom auf 15 l/h reduziert und das Reaktionsgemisch innerhalb einer Stunde auf Siedetemperatur erhitzt. Der Ansatz wird nun weiter gerührt wobei im Verlauf von 2,5 Stunden der Feststoff vollständig in Lösung geht. Nach weiteren 3 Stunden wird der Phosgenstrom abgestellt, Stickstoff eingeleitet, überschüssiges Phosgen ausgeblasen und das Lösungsmittel bei 15 mbar abdestilliert. Das Rohprodukt wird bei einem Vakuum von 1 mbar rasch destilliert und gaschromatographisch analysiert. Es werden 181 g Rohisocyanat erhalten, das zu 86,8% (70,6% der Theorie) aus 2,4,4'-Triisocyanatodicyclohexylmethan, 9,0% Diisocyanatodicyclohexylmethan und 4,2% aus unbekannten Produkten besteht. Nach Redestillation bei 180 bis 185°C/0,2 mbar werden 162 g Isocyanat isoliert das 95,5% 2,4,4'-Triisocyanatodicyclohexylmethan und 4,5% Diisocyanato-dicyclohexylmethan-Isomere enthält. Der NCO-Gehalt beträgt 39,0% und die Viskosität bei 25°C 109 mPa.s.

**Patentansprüche**

1. Gegebenenfalls Stellungs- und/oder Stereoisomerengemische darstellende Triisocyanate der Formel (I)

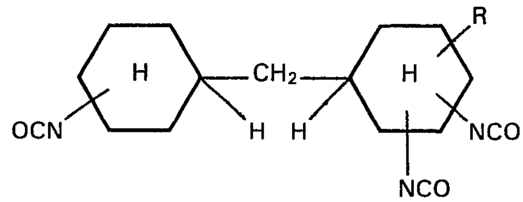

(I)

im welcher

R für Wasserstoff oder einen Alkylrest mit 1 bis 4 Kohlenstoffatomen steht.

2. Verfahren zur Herstellung von Triisocyanaten gemäss Anspruch 1, dadurch gekennzeichnet, dass man die ihnen zugrundeliegenden aromatischen Triamine in an sich bekannter Weise phosgeniert.

3. Verwendung der Triisocyanate gemäss Anspruch 1 als Aufbaukomponente bei der Herstellung von Polyurethankunststoffen nach dem Isocyanat-Polyadditionsverfahren.

**Claims**

1. Triisocyanates, optionally representing mixtures of position and/or stereo isomers, of the formula (I)

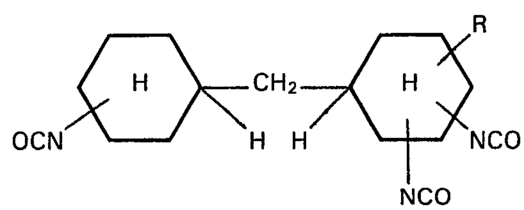

(I)

in which

R represents hydrogen or an alkyl radical with 1 to 4 carbon atoms.

2. Process for producing triisocyanates according to Claim 1, characterized in that the aromatic triamines on which they are based are phosgenated by methods known per se.

3. Use of the triisocyanates according to Claim 1 as a synthesis component in the production of polyurethane plastics by the isocyanate-polyaddition process.

**Revendications**

1. Triisocynates représentant éventuellement des mélanges d'isomères de position et/ou de stéreoisomères, de formule (I)

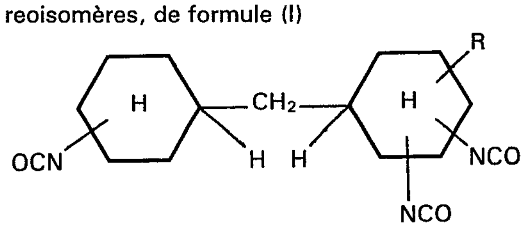

(I)

dans laquelle

R représente l'hydrogène ou un reste alkyle ayant 1 à 4 atomes de carbone.

2. Procédé de production de triisocyanates suivant la revendication 1, caractérisé en ce qu'on phosgène d'une manière connue les triamines aromatiques sur lesquelles ils sont basés.

3. Utilisation des triisocyanates suivant la revendication 1, comme composant structural dans la production de matières plastiques du type polyuréthanne selon le procédé de polyaddition d'isocyanate.